# EUROPEAN PATENT SPECIFICATION

(11) **EP 1 066 410 B1**
(45) Date of publication and mention of the grant of the patent: **20.10.2004**
(21) Application number: 99911947.2
(22) Date of filing: 24.03.1999
(51) Int. Cl.: C12Q 1/68

(54) **AMPLIFICATION OF NUCLEIC ACIDS**
VERVIELFÄLTIGUNG VON NUKLEINSÄUREN
AMPLIFICATION D'ACIDES NUCLEIQUES

(30) Priority: 25.03.1998 GB 9806253
(43) Date of publication of application: 10.01.2001
(73) Proprietor: TEPNEL MEDICAL LIMITED, Altrincham WA14 5UA (GB)
(72) Inventor: MULROONEY, Conor, Cheadle Hulme, Cheshire SK8 5DL (GB); OULTRAM, John Douglas, Manchester M41 6QB (GB)
(74) Representative: Atkinson, Peter Birch
(86) International application number: PCT/GB1999/000929
(87) International publication number: WO 1999/049081

(56) References cited:
- EP-A- 0 497 272
- EP-A- 0 500 224
- WO-A-94/16090
- WO-A-97/04126
- WALKER G T ET AL: "STRAND DISPLACEMENT AMPLIFICATION - AN ISOTHERMAL, IN VITRO DNA AMPLIFICATION TECHNIQUE" NUCLEIC ACIDS RESEARCH, vol. 20, no. 7, 1 January 1992 (1992-01-01), pages 1691-1696, XP002019521 ISSN: 0305-1048

### SEQUENCE LISTING

<110> TEPNEL MEDICAL LIMITED
<120> AMPLIFICATION OF NUCLEIC ACIDS
<130> TEPNEL PCT ISO-S
<140> PCT/GB99/00929
   <141> 1999-03-24
<150> 9806253.2
   <151> 1998-03-25
<160> 11
<170> PatentIn Ver. 2.0
<210> 1
   <211> 36
   <212> DNA
   <213> Artificial Sequence
<220>
   <223> Description of Artificial Sequence:PRIMER EDA M2 USED TO AMPLIFY A CONSERVED 129 BP REGION OF THE GLYCOPROTEIN B GENE OF HUMAN CMV
<400> 1
   tggtgtgtgg atcaacggcg caccctagag gtcttc                    36
<210> 2
   <211> 38
   <212> DNA
   <213> Artificial Sequence
<220>
   <223> Description of Artificial Sequence:PRIMER EDA M2 USED TO AMPLIFY A CONSERVED 129 BP REGION OF THE GLYCOPROTEIN B GENE OF HUMAN CMV
<400> 2
   acatcaccca tgaaacgcgc ggcaatcggt ttgttgta 38
<210> 3
   <211> 15
   <212> DNA
   <213> Artificial Sequence
<220>
   <223> Description of Artificial Sequence:PRIMER EDA D1 USED TO AMPLIFY A CONSERVED 129 BP REGION OF THE GLYCOPROTEIN B GENE OF HUMAN CMV
<400> 3
   tggtgtgtgg atcaa 15
<210> 4
   <211> 16
   <212> DNA
   <213> Artificial Sequence
<220>
   <223> Description of Artificial Sequence:PRIMER EDA D2 USED TO AMPLIFY A CONSERVED 129 BP REGION OF THE GLYCOPROTEIN B GENE OF HUMAN CMV
<400> 4
   acatcaccca tgaaac 16
<210> 5
   <211> 120
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 5
   tggtgtgtgg atcaacggcg caccctagag gtcttccaag gaactcagca agatcaaccc 60
   gtcagccatt ctctcggcca tttacaacaa accgattgcc gcgcgtttca tgggtgatgt 120
<210> 6
   <211> 35
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 6
   cgttcatcca tagttgcctg actccccgtc gtgta 35
<210> 7
   <211> 35
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 7
   tattgctgat aaatctggag ccggtgagcg tgggt 35
<210> 8
   <211> 16
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 8
   cgttcatcca tagttg 16
<210> 9
   <211> 16
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 9
   tattgctgat aaatct 16
<210> 10
   <211> 20
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 10
   ccctcccgta tcgtagttat 20
<210> 11
   <211> 18
   <212> DNA
   <213> Artificial Sequence
<220>
   <223> Description of Artificial Sequence:PRIMER USED TO AMPLIFY A 129 BP REGION BETWEEN BASES 1568 AND 1662 OF PUC19 BY PCR
<400> 11
   gtatcattgc agcactgg                                    18

## Description

The present invention relates to the amplification of nucleic acids, i.e. procedures for producing copies of nucleic acid sequences.

As used herein the term "nucleic acid" includes protein nucleic acid (PNA) (i.e. nucleic acids in which the bases are linked by a polypeptide backbone) as well as the naturally occurring nucleic acids (e.g. DNA and RNA), or analogues thereof, having a sugar phosphate backbone.

Various nucleic acid amplification techniques are already known, e.g. the Polymerase Chain Reaction (PCR). However many of these techniques (including PCR) suffer from the disadvantage that various cycles of heating and cooling are required for each amplification reaction. Thus, in a typical amplification reaction, the sequence (in single stranded form) to be amplified is treated with an oligonucleotide capable of hybridising to the sequence at a particular location thereof, the treatment being effected at a temperature (and under other conditions, e.g. buffers etc.) at which the hybridisation will occur. In the next step (which may or may not be effected at the same temperature) a polymerase enzyme is used to extend the oligonucleotide primers (using the original sequence as a template) to produce a strand which is complementary to the original strand and which is hybridised thereto. Subsequently the reaction mixture must be heated to denature the complementary strands and then cooled so that the above described procedure (i.e. primer hybridisation, extension, denaturing) may be repeated. A particular amplification reaction may require many repeats of the cycle before a sufficient quantity of the nucleic acid is generated (e.g. for the purposes of a diagnostic test or for research purposes). The need to "temperature cycle" the reaction many times is a considerable disadvantage.

Several alternative amplification strategies are known which can be performed at a single temperature. Examples of such isothermal technologies include Strand Displacement Amplification (SDA), (G. T. Walker; US Patent No. 5455166).

SDA takes advantage of the ability of certain restriction enzymes to cleave hemi-modified restriction sites only on the un-modified DNA strand. By using non-modified primers containing such a restriction site, and including modified dNTP analogues, it is possible to introduce single strand nicks into the primer when it is rendered double stranded by DNA which has been synthesised to include modified nucleotides. The introduction of this nick allows a strand displacing polymerase to extend the sequence upstream of the nick by displacing downstream sequences into solution where they are picked up by primers directed to the other end of the strand to be amplified. The process is rapid and can be performed at a single temperature. However, SDA requires specialised primers, which are not wholly specific to the natural target DNA sequence to which they are directed. This arises from the need to introduce into the sequence to be amplified restriction sites at the ends of the molecule for the reaction to occur. Furthermore, a degree of manipulation is required to initiate the amplification reaction. For instance, a second set of primers are required to displace the extended first copies into solution so that the second amplification primer can act upon the single strand, displaced, extension product.

Another isothermal amplification method known in the art is Exonuclease Mediated Strand Displacement Amplification (G. T. Walker, EP-A-0 500 224). This technique takes advantage of the ability of thiol-substituted nucleotide analogues to protect downstream sequences from digestion by a 5' double strand specific exonuclease. In this method unmodified primers are used together with a 5' double strand specific exonuclease, a strand displacing DNA polymerase and an excess of deoxynucleosidetriphosphates, at least one of which is substituted to provide resistance to exonuclease activity. The method as described, however, will not provide for exponential amplification as the loss of unmodified primer sequences (by exonuclease activity) precludes the copying of those same sequences, to allow hybridisation at the complementary sequence, later in the reaction

In another amplification method (P. Cleuziat, WO-A-97/04126) chimeric RNA\DNA primers are used, together with shorter DNA primers, a strand displacing polymerase, an excess of deoxynucleosidetriphosphates and RNAase H. Binding of the chimeric primer to its target generates a short, double-stranded, DNA/RNA hybrid from which the RNA portion is cleaved by the action of RNAse H. This reveals a site for the shorter DNA primer which hybridises and is extended by the action of the DNA polymerase..A disadvantage of this system is the need for the RNA carrying primers. RNA is notoriously sensitive to degradation by ribonucleases which are abundant contaminants of many environments, and, extensive decontamination procedures must be observed to limit this effect during RNA manipulations. Because of the low turnover rate of the RNase H enzyme, longer reaction times must be used or the enzyme must be present at a higher concentration. Also, the amplification must be performed in the abiding presence of non-target sequences, present in the sample, which are known to contribute to anomalous results during other amplification reactions, such as PCR.

It is an object of the present invention to provide an amplification method which may be effected under isothermal conditions, if required, and can amplify the natural sequence entirely, if required.

According to the present invention there is provided a method of amplifying complementary first and second nucleic acid sequences each of which has a binding region at its 3' end, the method comprising treating the separated single stranded sequences with
(a) first and second primers each capable of hybridising to the 3'-binding regions of the first and second strands respectively and each including remote from its 5'-end a digestion resistant region which, with the primer hybridised to the complementary 3'-binding region, allows only partial digestion of the primer by the enzyme (d) having 5'-double strand specific exonuclease activity,
(b) third and fourth primers each having a degree of sequence homology with the partially digestible regions of the first and second primers respectively whereby the third and fourth primers are capable of hybridising to the 3'-binding regions of the first and second strands respectively,
(c) an enzyme having strand displacing polymerase activity,
(d) an enzyme having 5' double stranded specific exonuclease activity, said enzyme (d) possibly being provided by enzyme (c) in the case where the latter also has the required exonuclease activity, and
(e) nucleoside triphosphates,
under conditions permitting hybridisation, exonuclease digestion and strand displacement polymerisation thereby producing an amplified amount of the first and second strands.

As used herein, the term 5 -double stranded specific exonuclease activity means that the principal exonuclease activity is double stranded activity whilst not precluding the possibility of a degree of single stranded activity. The term 5'-double stranded specific exonuclease is to be interpreted accordingly.

The present invention thus provides a method for amplification of a nucleic acid molecule. The complementary sequences on which the reaction is effected may be generated (e.g. *in situ* in the reaction) from a single stranded molecule by methods well known in the art.

The method may be effected under conditions such that all of reactants (a)-(e) are simultaneously present in a reaction mixture or under conditions in which the reagents are used sequentially as appropriate.

The method utilises a 5' double stranded specific exonuclease, i.e. an exonuclease which will digest the strands of a double stranded molecule from the 5' ends thereof. The method also requires the use of an enzyme having strand displacing polymerase activity. The method of the invention may be performed with two enzymes, one being a 5' double stranded specific exonuclease and the other being a strand displacing polymerase. It is however also possible to use a single enzyme having both of these activities.

The invention further utilises the first and second primers each of which incorporates a region (e.g. provided by modified nucleotides, see below) resistant to digestion by the 5' double stranded specific exonuclease. It should however be understood that the terms "first" and "second" primers are used in an explanatory sense to identify primers which hybridise to the 3' binding regions of the first and second strands respectively. The terms do not necessarily imply that the primers are different since it is possible to envisage embodiments of the invention in which the 3' binding regions of the first and second strands have sufficient sequence identity for the "first" and "second" primers to be one and the same.

The method further utilises the third and fourth primers which need not incorporate modified nucleotides and which have a degree of sequence homology with the partially digestible regions of the first and second primers. In certain embodiments of the invention, the "third" and "fourth" primers may be identical to each other.

A further description as to the "mechanism" by which the amplification occurs is given below with reference to the drawings.

The digestion resistant regions of the first and second primers may comprise at least one modified nucleotide resistant to digestion by the 5' double stranded specific exonuclease. It is a requirement that the digestion resistant region is not wholly digested by the 5' double stranded specific exonuclease. This may be achieved by appropriate selection of the type and number of modified nucleotides provided in digestion resistant region. It will generally be preferred that the region incorporate several (e.g. at least 4) modified nucleotides to ensure that the region is not totally digested. The modified nucleotides may for example be thiolated or boronated nucleotides or indeed be ribonucleotides. It is preferred that the first and second primers include at least one (e.g. preferably four and more preferably six) internal phosphorothioate linkages.

It is preferred that each of the first and second primers has 30 to 60 bases and that the third and fourth primers have about half the number (e.g. 12 to 30) of bases as the first and second primers. The digestion resistant region may be provided 15 to 25 bases from the 5' ends of the first and second primers. Each of the third and fourth primers will for preference be of a sequence corresponding to those regions of the first and second primers respectively which are 5' to the 5' end of the digestion resistant regions thereof. However we do not preclude the possibility of the 3' ends of the third and fourth primers incorporating bases corresponding to some of the modified nucleotides provided at the 5' ends of the digestion resistant region (of the first and second primers) to the extent that these modified nucleotides are likely to be digested during partial digestion of the region.

It is particularly preferred that the polymerase which is used is one which has activity at the same temperature as that at which the 5' specific double strand specific exonuclease has activity whereby the reaction may be effected isothermally. For preference this temperature is 37°C.

The 5' double strand specific exonuclease may for example be T7 gene 6 exonuclease.

The strand displacing polymerase may for example be at least one of 9°N polymerase, Klenow (exo⁻) polymerase, *Bst* polymerase, Vent (exo⁻) polymerase, Deep Vent (exo⁻) polymerase, *Pfu* (exo⁻), *Tth, Tfl, Taq* or *Bca* (exo⁻) polymerase.

The nucleotides as identified above as (d) of the reaction mixture may be dATP, dCTP, dGTP and dTTP. It is however possible for at least a portion of at least one of these nucleotides to be replaced by a substituted nucleotide whereby a strand (generated during the reaction) incorporating the modified nucleotide is resistant to digestion by the 5' double strand specific exonuclease.

It is an advantage of the invention that the reaction may be effected without pre-manipulation (e.g. restriction digestion) of the target to be amplified, such manipulations complicating other methods known in the art. It is further advantage that non-amplifiable sequences, which might otherwise adversely affect the reaction (e.g. non-target nucleic acid, non-protected primer dimers), are degraded during the amplification process.

The invention will be further described by way of example only with reference to the accompanying drawings, in which:
Fig. 1 schematically illustrates a DNA molecule for amplification in accordance with the method of the invention;
Fig. 2 schematically illustrates four oligonucleotide sequences (primers) for use in forming an amplified product from the molecule shown in Fig. 1;
Fig. 3 schematically illustrates one embodiment of the method of the invention;
Fig. 4 schematically illustrates a second embodiment of the method of the invention; and
Figs. 5-6 illustrate the results of the examples.

In the following description, sequences which are complementary to each other are denoted by the same letters (or sequence of letters) but with one of the two complementary sequences additionally being denoted by the "prime" suffix ('). Thus X' is the complementary sequence to X.

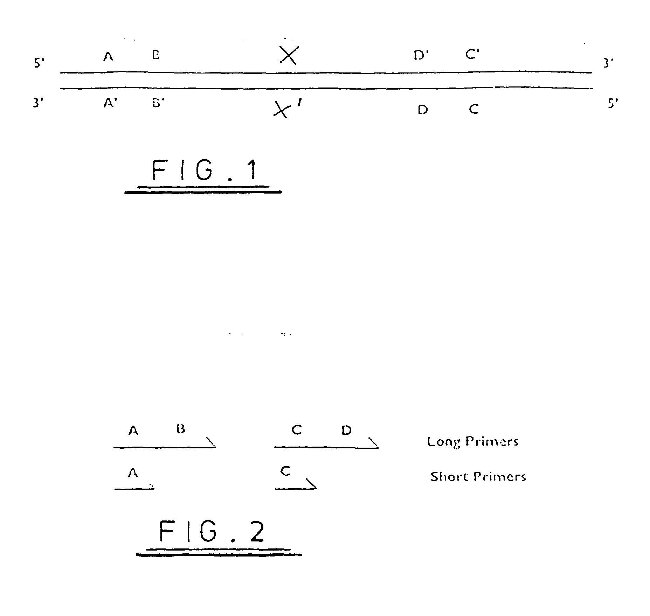

Fig. 1 illustrates a double stranded molecule incorporating a sequence X and its complement X' to be amplified by the method of the invention.

For convenience in the following description, the sequence X is shown as having a 3' binding region denoted by D'C'. Furthermore, sequence X' is shown as having a 3' binding region B'A' (adopting the convention that a segment of the nucleic acid molecule is annotated from the 5' end of the molecule). Binding sequences D'C' and B'A' may for example each comprise 30-60 bases of which sequences A and C may each provide about half of the number of these bases. Thus for example sequences A and C may each provide 12-30 bases

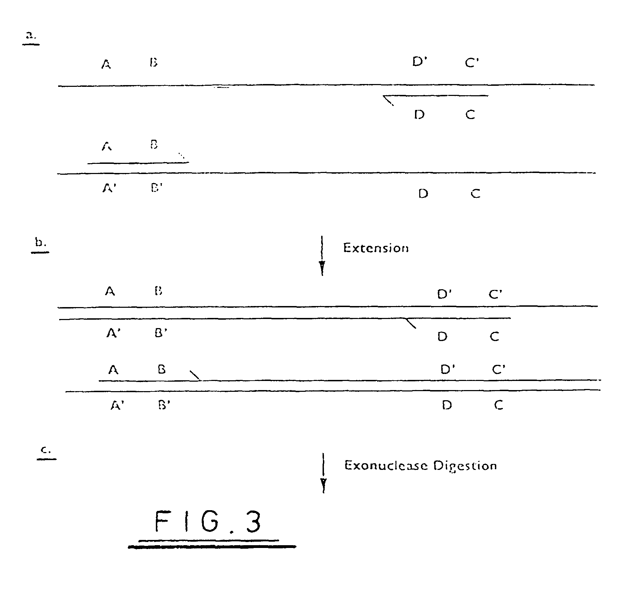

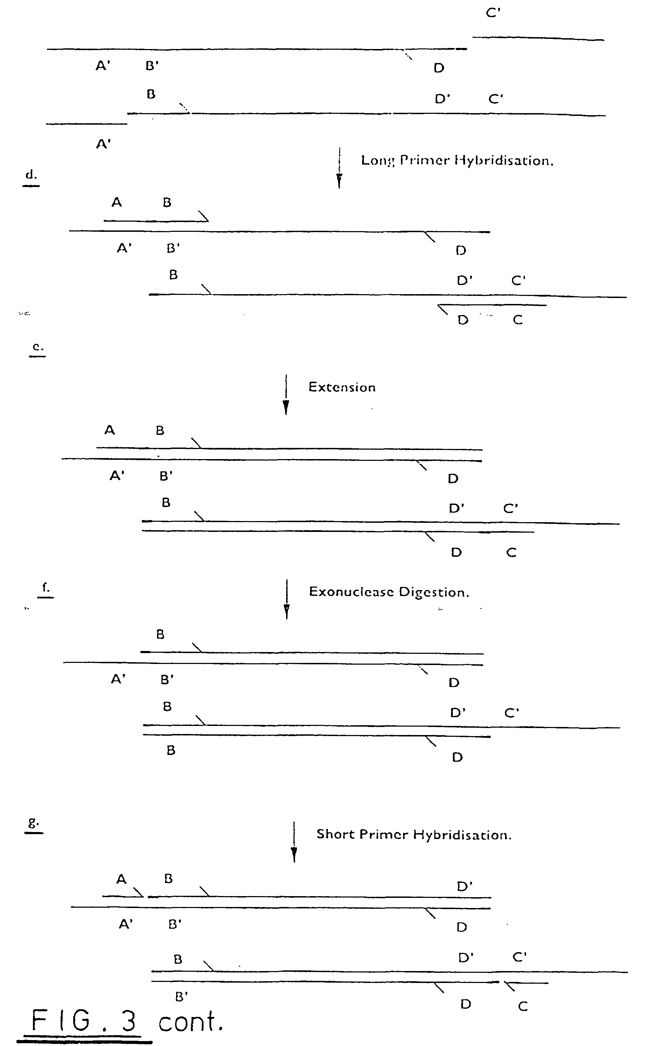

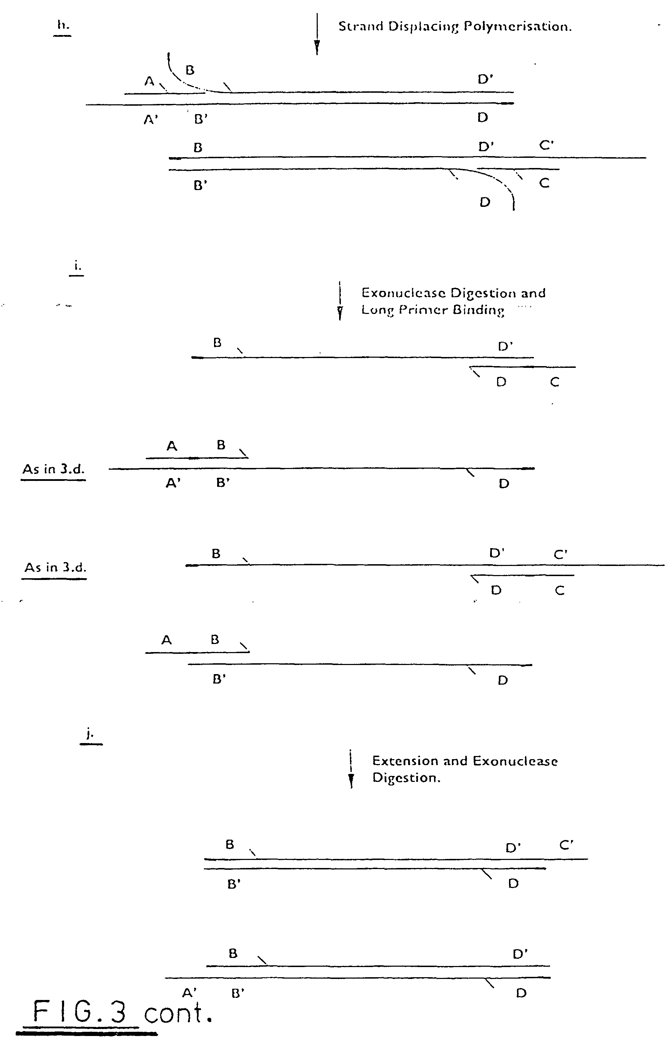

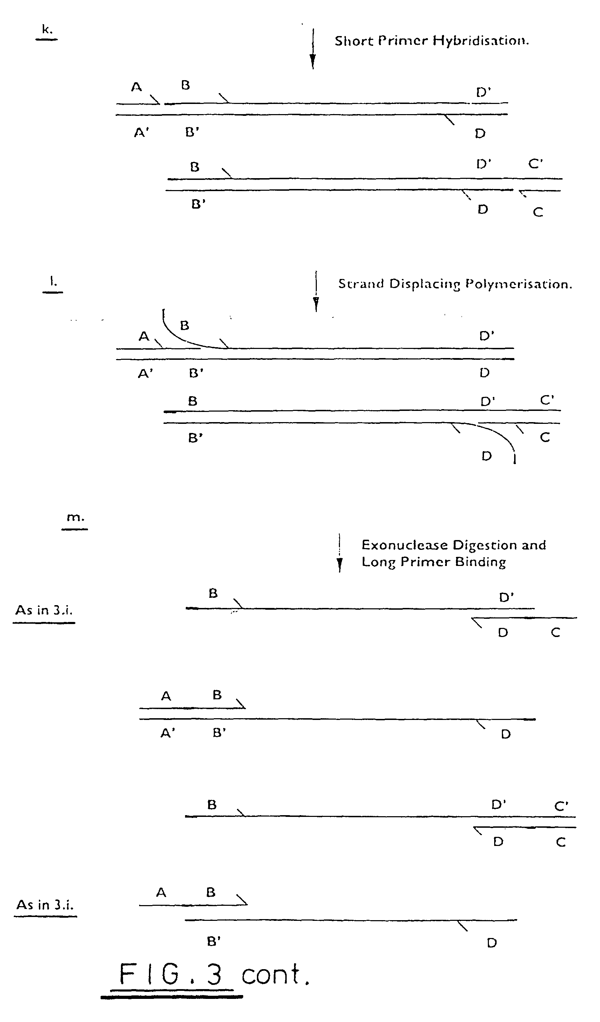

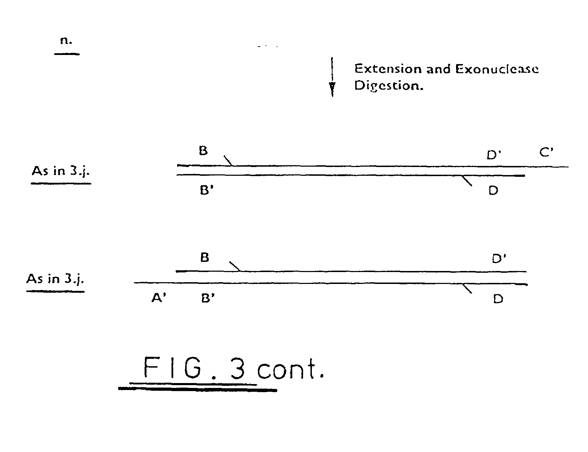

Referring now to Fig. 2, there is illustrated therein four primers which are to be used in the method to be described more fully below with reference to Fig. 3. More particularly, the primers shown in Fig. 2 comprise first and second primers AB and CD respectively (also designated herein as "long" primers) together with third and fourth primers A and C respectively (also designated herein as "short" primers).

The first primer AB is capable of hybridising to the 3' binding region B'A' associated with sequence X' to be amplified. Similarly the second primer CD is capable of hybridising to the 3' binding region D'C' of the sequence X to be amplified. As such, each of primers AB and CD may comprise 30 to 60 bases. Furthermore primer AB incorporates a digestion resistant region (denoted by the thickened line) which is comprised of at least one and preferably a plurality of modified nucleotides so as to be resistant to digestion by a 5' double strand specific exonuclease used in the reaction described more fully below and which is located between sequences A and B. Similarly the primer CD also includes a digestion resistant region between sequences C and D.

The third and fourth primers each comprise sequences A and C respectively and do not incorporate modified nucleotides.

Reference is now made to the reaction depicted in Fig. 3 which takes place in the presence of the strands to be amplified, nucleotides, a strand displacing polymerase, a 5' double strand specific exonuclease and appropriate buffers.

For simplicity, Fig. 3 does not show any hybridisation/extension reactions which would result in production of a double stranded nucleic molecule not incorporating a digestion resistant region since such double stranded molecules would be digested by the 5' double strand specific exonuclease and not contribute to the overall result of the reaction.

To effect the reaction shown in Fig. 3, the double stranded target molecule shown in Fig. 1 is manipulated, by methods known in the art, so as to be in single stranded form as depicted in Fig. 3a. In this form, the first primer AB hybridises to the binding region B'A' and primer CD hybridises to binding region D'C'. Extension of primers AB and CD by the polymerase generates the two partial double stranded products illustrated in Fig. 3b. The double stranded molecules are the converted to the products of step (d) by one of two mechanisms.

In one mechanism the double stranded regions of the products serve as substrate for the 5 double strand specific exonuclease which removes those regions not protected by upstream (5') digestion resistant region, generating the single strand products shown in Fig.3.c.

Long primers then hybridise to their complementary sequences in the single stranded products of step (c) (Fig.3.d.).

The other, and possibly more likely, mechanism is similar to that shown below with reference to steps (g) and (h). More particularly, the 5'-ends of the extension products the long primers AB and CD are digested by the exonuclease to reveal sites to which the appropriate short primers A and C may bind. These short primers then undergo strand displacing polymerisation resulting in the production of single stranded molecules to which the long primers AB and CD as appropriate may bind (i.e. resulting in the products of step (d)).

The hybridised long primers in the products of step (d) are extended by the above polymerase to generate the two partially double stranded products shown in Fig.3.e. In these products the only region that is susceptible to attack by exonuclease are the unprotected 5' regions of the extended Long Primers. These regions are, thus, removed by action of the exonuclease to generate the products seen in Fig.3.f.

The single strand 3' overhanging regions created by the action of the exonuclease contain hybridisation sites for the short primers. Fig. 3.g. illustrates the hybridisation of Short Primers at these sites. The Short Primers are so positioned as to allow their extension by a 'strand displacing' DNA polymerase with the concurrent displacement of the downstream target copy into solution, as shown in Fig.3.h. The extended strand produced by strand displacing polymerisation of the Short Primer is not protected by nucleotide modification and will be degraded by the action of exonuclease to leave a single stranded product to which the appropriate Long Primer can adhere. Simultaneously, the displaced single strands contain, at their 3 ends, sites for hybridisation of the 3' ends of yet further Long Primers.

The four molecules, which result from strand displacing polymerisation, exonuclease degradation of susceptible double stranded regions, and hybridisation of suitable primers, is shown in Fig. 3.i.. Two of these molecules, those produced by exonuclease degradation of the unprotected Short Primer extension products, are functionally identical to those generated in Fig.3.d. and, in suitable reaction conditions, will continue to cycle through the steps described between Fig.3.d. and Fig.3.i with the production of the two extra products seen in Fig. 3.i., at each cycle, and will not be considered further in the description. The two 'extra' products shown in Fig.3.i. are further processed, firstly, by the action of DNA polymerase, which will generate double stranded molecules. The long Primers from which polymerisation occurred are partially susceptible to exonuclease degradation once they are rendered double stranded. The results of extension and exonuclease digestion of the susceptible regions are shown in Fig.3.j. As before, the digestion of the Long Primer by exonuclease reveals a site at which the short Primer will hybridise, generating the molecules seen in Fig.3.k.. Strand Displacing Polymerisation of the Short Primers (Fig. 3.1.), followed by exonuclease digestion of the extended strand, will again generate four molecules to which the Long Primer can hybridise, as shown in Fig.3.m.

The first and fourth products shown in Fig. 3.m. are functionally identical to the products shown in Fig.3.i. and, in the continuance of suitable reaction conditions, will cycle through the steps described between Fig.3.i. and Fig.3.m. with the production of the two extra products seen in Fig. 3.m., at each cycle, and will not be considered further in the description.

The second and third products shown in Fig.3.m. are similar to those shown in Fig.3.d. except the molecule to which the long Primer is hybridised is bounded, at both ends, within the region to be amplified. Extension of the Long Primer by polymerase and exonuclease digestion of the susceptible region double strand produced generates the products shown in Fig.3.n., which are functionally identical to those shown in Fig.3.j., and, in the continuance of suitable reaction conditions, will cycle through the steps described between Fig.3.j. and Fig.3.n, with the production of the two extra products seen in Fig. 3.m., at each cycle.

A number of modifications may be made to the process illustrated in Fig 3. For example, the 5 digestible regions of the first and second primers may be identical and such that they do not hybridise to the original target molecule (whereby the first and second primers only partially hybridise thereto). In this case, the first and second primers give rise to extension products having identical sequences of their 3 ends this permitting the third and fourth primers to be identical. There is the additional advantage that the use of a short sequence (in the first and second primer) for hybridising to the original target molecule provides for higher stringency.

Alternatively or additionally the 5'-ends of the first and second primers may have a degree of resistance to digestion (whilst still leaving a "digestible" region of the primer) to slow down the rate at which the primer is digested in the event that the 5'-double strand specific exonuclease also has activity for digesting single strands. Clearly however this 5-ends of these primers should not be totally resistant to digestion. The partial degree of resistance may be provided by one or more nucleotides.

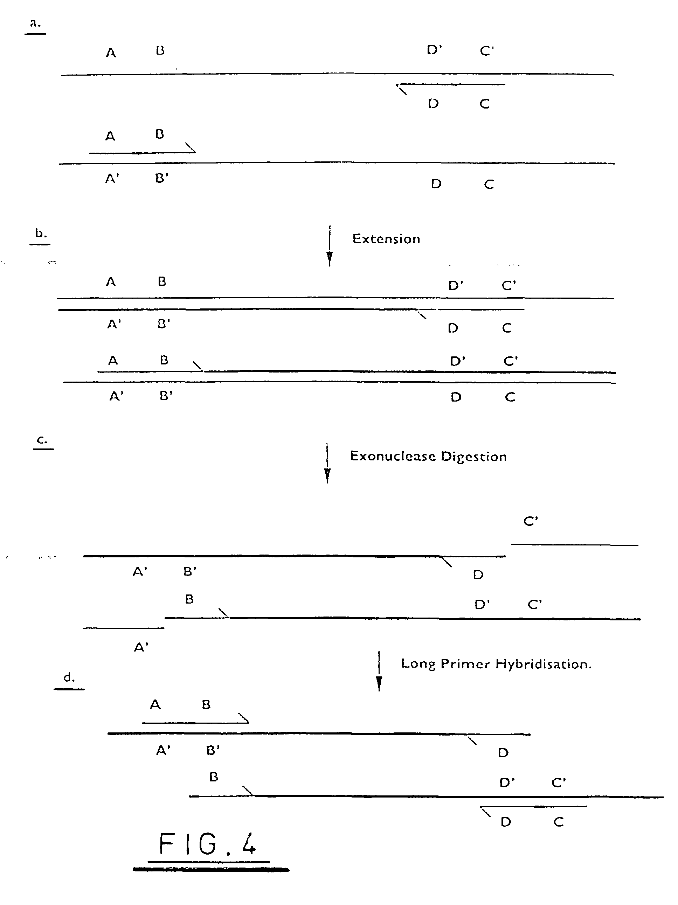

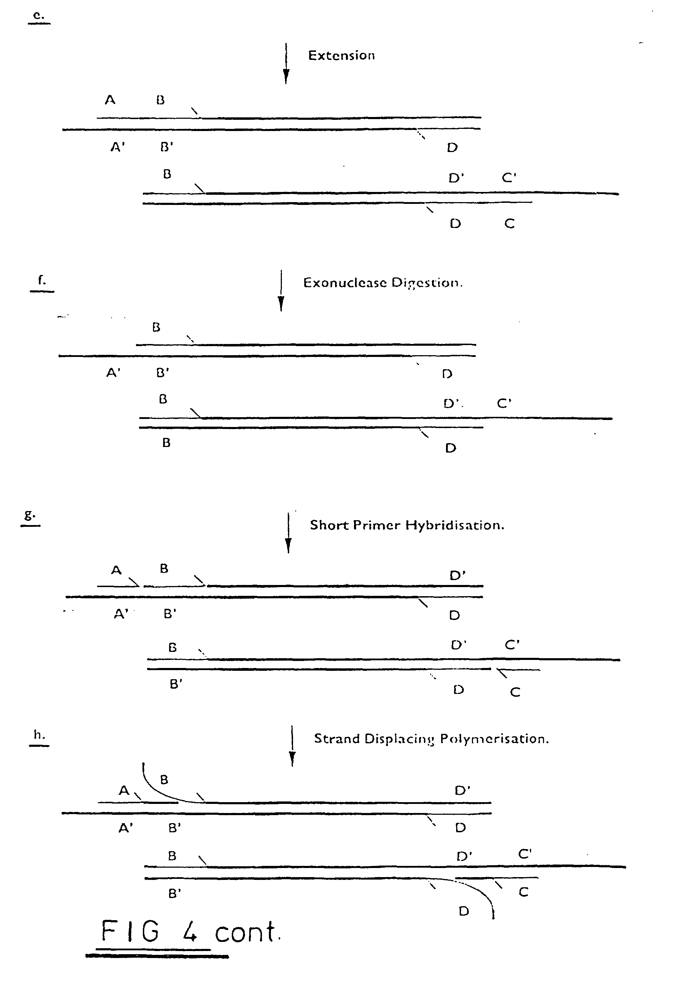

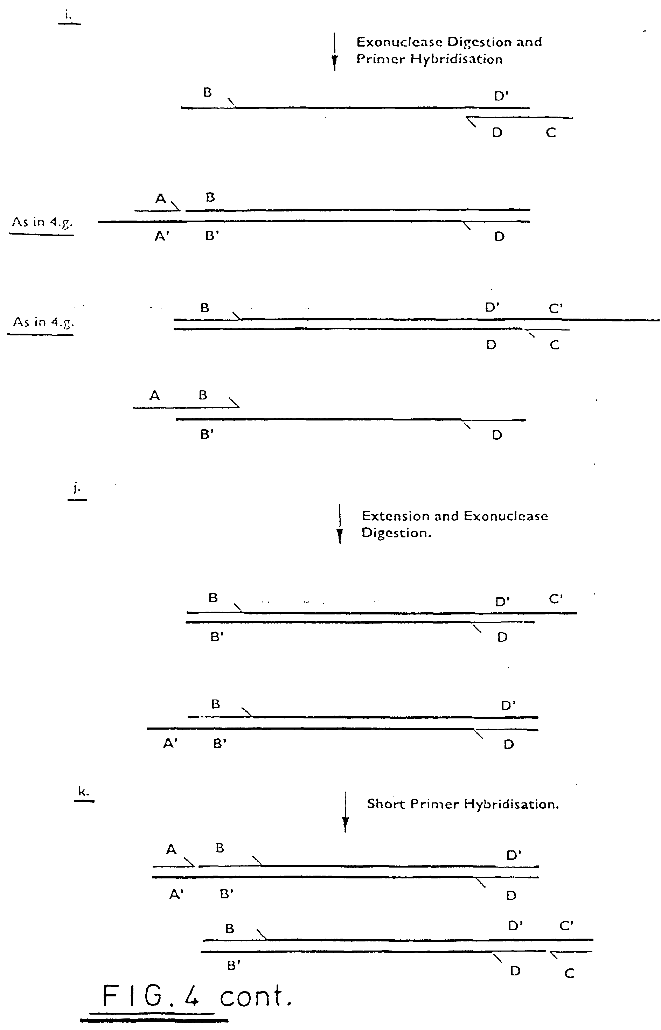

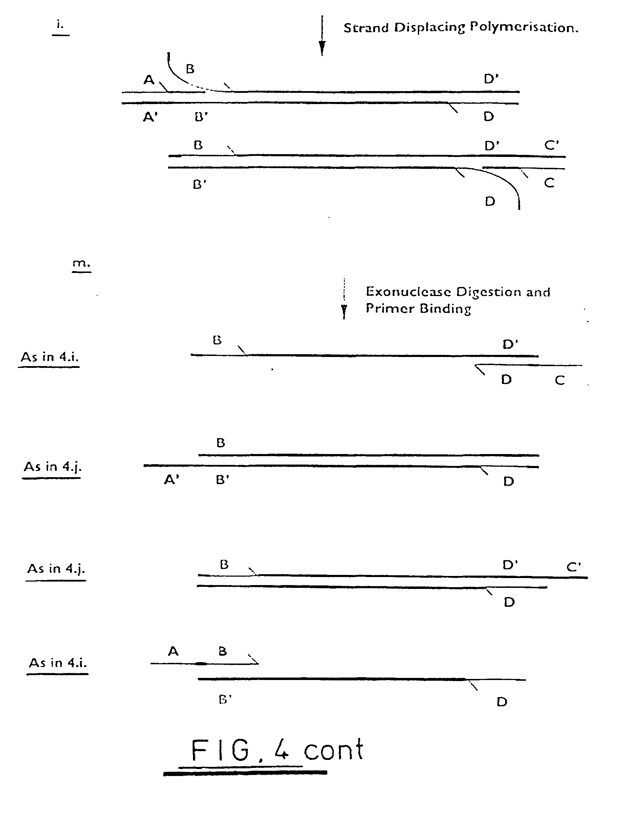

Figure 4. illustrates a second embodiment of the method, which differs from the method outlined in Figure 3. by the inclusion in the reaction of modified nucleosides, which, upon incorporation into an extension product, provide some protection of downstream sequences from attack by double-strand specific 5' exonuclease.

Fig. 4.a. shows the target molecule of Fig.2. rendered single stranded, with the Long Primers of Fig. 1. hybridised thereto. Fig. 4.b. shows the product of DNA polymerase mediated extension from the hybridised primers, using the target molecule as template. The thickened lines in Fig. 4. illustrate those regions which contain modified nucleotides and are, therefore, refractant to digestion by the double-strand specific 5' exonuclease.

The next figure (Fig.4.c.) illustrates the products of exonuclease digestion of the extended products of Fig. 4.b. Exonuclease digestion reveals sites, on the newly synthesised strand, for the primers of the opposite pair to that used in its creation. Fig. 4.d. illustrates the product of Long Primer hybridisation at these sites.

Fig.4.e. illustrates the products of extension of the newly hybridised primers by the DNA polymerase using the synthesised target copy as template and incorporating nucleotides so modified as to render downstream (3') sequences resistant to exonuclease attack.

Fig. 4.f. illustrates the products of the action of double-strand specific 5' exonuclease on the susceptible regions of the products shown in Fig. 4.e. Such action reveals hybridisation sites for the Short Primers of Fig. 2., and Fig. 4.g. shows the products of hybridisation of the short primers onto these.

Fig. 4. h. illustrates the extension of the attached Short Primer sequences, using the target copy as template, by the 'strand displacing' polymerase, with concomitant displacement of the downstream sequence into solution. The extending Short Primer incorporates the modified nucleotides such that the newly synthesised region is refractant to the action of double-strand specific 5 exonuclease. The products of the strand displacing polymerisation are, therefore, resistant to exonuclease attack, except for the unprotected sequence of the Short Primer itself, which will therefore be removed. Removal of the Short Primer sequence will regenerate the site for Short Primer hybridisation. The second and third products of Fig. 4.i. illustrate the products of Short Primer hybridisation to these exposed sequences. It can be seen that these products are functionally identical to the products shown in Fig.4.g. and, in the continuance of suitable reaction conditions, will cycle through the steps described between Fig.4.g. and Fig.4.i. with the production of the two extra products seen in Fig. 4.i., at each cycle. These products will not, therefore, be considered further in the description.

The first and fourth products shown in Fig.4.i. illustrate the single stranded displacement products of Fig. 4.h. with, hybridised at their 3' ends, the 3' ends of Long Primer sequences. Extension from these sequences, incorporating 'modified' nucleotides, and exonuclease digestion of vulnerable regions of the double stranded products of such extension, will yield the products shown in Fig. 4.j.. These products have short, single stranded, regions at one end, which are complementary to, and hence hybridisation sites for, the Short Primers.

Fig. 4.k. illustrates hybridisation of Short primers at their hybridisation sites.

Fig. 4.l. illustrates strand displacing polymerisation of the hybridised Short Primer sequences, using the target copy as template, incorporating modified nucleotides to protect newly synthesised sequences from exonuclease digestion.

Fig. 4.m. illustrates the products of the strand displacing polymerisation shown in Fig. 4.l. following exonuclease digestion of susceptible regions and hybridisation of primers at potential hybridisation sites. The displaced single strands (products one and four shown) contain sites for hybridisation of Long Primers and are then functionally identical to the products shown in Fig 4.i.. In the continuance of suitable reaction conditions, these products will cycle through the steps described between Fig.4.i. and Fig.4.m. with the production of the two extra products seen in Fig. 4.m., at each cycle.

Likewise, the other two products in Fig. 4.m. (the second and third products) are functionally identical to the products shown in Fig. 4.j. and, in suitable reaction conditions, will continue to cycle through the steps described between Fig.4.j. and Fig.4.m. with the production of the two extra products seen in Fig. 4.m., at each cycle.

For reasons of simplicity the methods outlined have been described in terms of discrete steps involving the actions of the of the enzymes involved running to completion before the next step in the process begins. While, in certain circumstances, this may be possible, by manipulation of conditions during the reaction such that only one or other of the enzymes involved is active at any given point in the reaction, in the preferred embodiment of the reaction it is envisaged that the reaction will occur in conditions which promote the simultaneous action of all components of the reaction.

It should be further noted that, in a number of the schematic steps, when hybridisation sites are produced, since Long and Short primers share sequence homology, either may come to occupy those sites and, furthermore, may not be able to take part in the reaction as described. In these cases processes other than those explicitly described may occur. These might include;
a) Short Primer occupying the hybridisation sites outlined in Fig3.a. d. i. (the second and third products) and m. (the second and third products). In these cases, the likely outcome is a fatuous extension of the primer, using the target copy as template, to give an unprotected extension copy, which will be removed, by the action of the exonuclease, to reveal the hybridisation site once more.
b) Short Primer occupying the hybridisation sites outlined in Fig. 4.a. and d.. In these cases the likely outcome is extension of the primer, using the target copy as template, and incorporating modified nucleotides such that the newly synthesised material is protected from digestion by the exonuclease. This product differs from the products described in the relevant figures only in the extent of modification of the sequence 3' to the short primer, which the Long Primer would have occupied. This should have little or no effect upon amplification and these products should become incorporated into the reaction in the same way as Long Primer products would.
c) Long Primer occupying the hybridisation sites outlined in Fig 3. g. and k. and Fig 4. g. i. (the second and third products) k. and m. (the second and third products). In these cases, the most likely outcome is the digestion of the unprotected 5' region of the Long Primer, which will cause it to dissociate from the hybridisation site, revealing the site once more.

While the above reactions may affect the efficiency of the amplification reaction, they do not alter the nature of the processes involved and will not be discussed further.

For the reasons outlined above, the schematic diagrams in Fig. 3. and Fig. 4. should be taken as indicative of the processes occurring, rather than an exact blueprint of the reaction.

Additional modifications/additions to the method may be made using techniques known in the art. These may include;
a) Using a non-natural target sequence such as a ligation product, a wholly synthesised sequence, a closed circular target molecule etc.
b) Using as a target RNA, either naturally derived or wholly, or partially, manufactured, which can be converted to a homologous or complementary DNA sequence by methods known in the art.
c) Using Long Primers which share 5' sequence homology such that a single Short Primer could serve to generate product from each end of the target molecule during the amplification reaction.
d) Using target sequences or one, or more, primers which are covalently, or non-covalently, attached to a solid phase.
e) The amplification may be effected in the presence of further primers specific to other target sequences so as to provide multiplex amplification or to all or some of the same target, sequence so as to effect amplification.

While the example reactions below are performed at 37°C using T7 gene 6 exonuclease and the exonuclease deficient variant of.the Klenow fragment of E. Coli DNA polymerase, there are polymerase enzymes known in the art which have higher temperature optima. In the presence of a thermophilic double strand specific exonuclease, the reaction should occur at elevated temperatures.

The invention will be illustrated by the following non-limiting examples.

### Example 1

An amplification reaction was carried out using the following primers, template and reaction conditions. Two sets of primers were used. The first were primers EDA M1 and EDA M2 (a 37-mer and a 38-mer) containing four internal phosphorothioate linkages in series. The second set were the short displacement primers (16-mers) EDA D1 and EDA D2 whose sequences were identical to the first 16 bp of the long primers reading from the 5' end. (Italicised bases indicate the location of the phosphorothioate linkages.)

The short primers were used to amplify a conserved 129bp region of the glycoprotein B gene of Human CMV by PCR. This was the template used in the Amplification reaction and due to the primers used, the PCR amplified fragment contained no phosphorothioate linkages 21bp or other means of protection.

The following reaction mixture was prepared in a 0.2 ml thin walled PCR tube:5µl of 10x Klenow (exo⁻) reaction buffer (500 mM Tris-HCl pH 7.5, 100mM MgCl₂ , 10 mM DTT, 0.5 mg/ml BSA [Amersham]), 2µl of 10mM dNTP's (Pharmacia), 150 pmoles each of primers EDA M1 and M2, approximately 120 pmoles of 129 bp template and ultrapure water to a final volume of 50µl. Samples were mixed and incubated at 96°C for three minutes to denature and then allowed to cool to 37°C for 1 minute before addition of 20 units of Klenow (exo-) polymerase. Incubation was allowed to proceed at 37°C for two minutes before addition of the following mixture: 120 pmol each of primers EDA D1 and D2, 15 units of T7 gene 6 exonuclease, 5µl of 10x Klenow (exo⁻) polymerase (Amersham), and ultrapure water to a final reaction volume of 50µl. 16µl aliquots were taken at the following intervals '0 time', 60, 90, 120, 150 and 180 minutes. These were mixed with an appropriate dilution of loading dye and stored at 4°C until ready for visualisation by electrophoresis on a 3% agarose gel containing ethidium bromide to a final concentration of 0.3µg/ml.

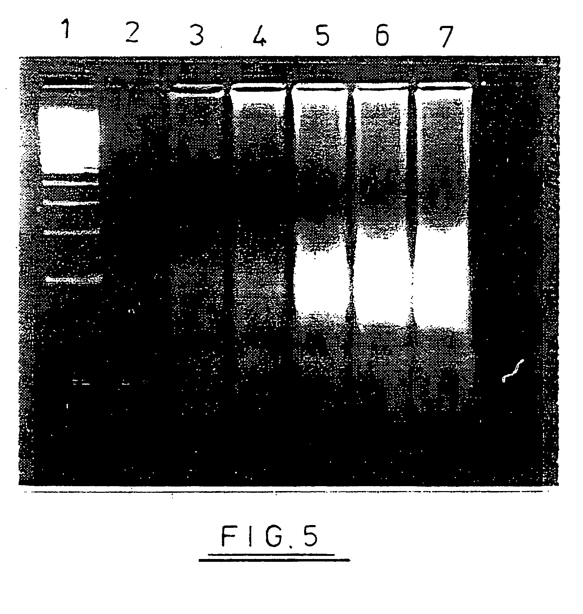

The results are shown in Fig. 5 in which lane is a 100bp marker and lanes 2-7 respectively illustrate the results obtained at "0 time" 60, 90, 120, 150 and 180 minutes. The production of amplified product is clearly shown in lanes 5-7.

### Example 2

An amplification reaction was carried out using the following primers, template, and reaction conditions. Two sets of primers were used. The first were long primers EDLM and EDUM (both 35mers) containing six internal phosphorothioate linkages in series. The second set were the short displacement primers (16-mers) EDUDP and EDLDP whose sequences were identical to the first 16 bp of the long primers and which contained two phosphorothioate linkages at their 5' end.

(Italiscised bases indicate the location of the phosphorothioate linkages. Bases in bold indicate the presence of biotin label. Primers EDUDP (batch 1139-1) and EDLDP (batch 1139-2) were supplied by MWG-Biotech UK, Milton Keynes. UK; primers EDLM and EDUM were synthesised at Tepnel Life Sciences UK).

The long primers were used to amplify a 129bp region (between bases 1568 and 1662) of pUC 19 by PCR. This was the template used in the EDA reaction and due to the primers used; the PCR amplified fragment contained phosphorothioate linkages 18 bp from the 5' terminus of both strands.

The following master mix was prepared: 15ml of x10 Klenow (exo-) reaction buffer;, 12ml of 10mM dNTP's; 6ml of 20 pmole/ml EDUM; 6ml of 20 pmole/ml EDUM; and ultrapure water to 120ml final volume. 10ml of ultrapure water containing 25ng, 1ng or 0ng of PCR amplified 129 bp pUC 19 template, was added to 40ml of the above master mix, in 0.2 ml thin walled PCR tubes These samples were heated to 98°C for 2 minutes on a Biometra Trio 48 thermocycler before addition of 50ml of a master mix comprising; 50ml of x10 Klenow (exo-) buffer, 4.5ml of 50 pmole/ml EDLDP; 4.5ml; 50 pmole/ml EDUDP ; 4.5m of 50 pmole/ml EDUDP1; 6ml; 5unit/ml Klenow (exo-) polymerase; 1.8ml of 50 units/ml T7 gene6 exonuclease; and ultrapure water to 150µl final volume.

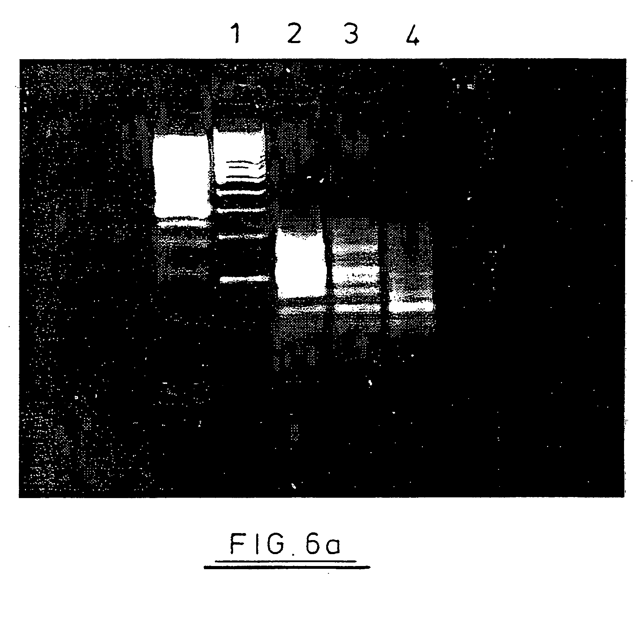

Samples were incubated at 37°C for 4 hours. 17µl aliquots were visualised on a visualised by electrophoresis on a 3% agarose gel containing ethidium bromide to a final concentration of 0.3µg/ml (Figure 6a). In Fig 6a, lane 1 is a 100 bp marker, and lanes 2-4 show the results obtained using 25ng, 1ng and 0ng of template respectively. The production of amplified product can be seen in lanes 2 and 3.

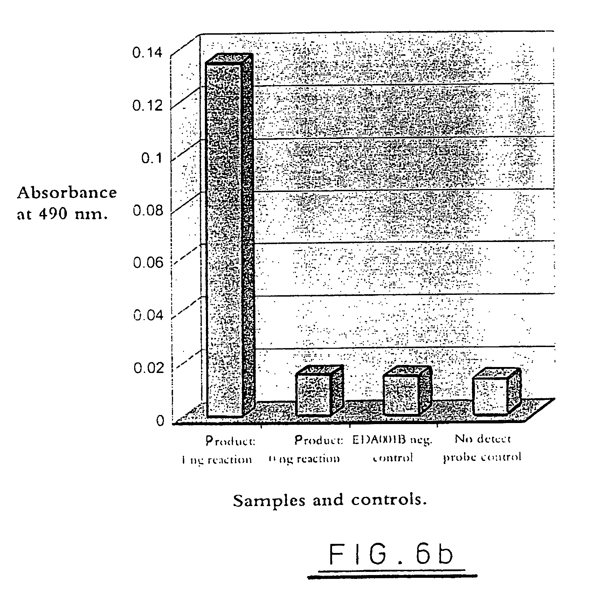

In addition, the products from the reactions containing 1 ng of template and 0 ng of template were tested using the DARAS™ system to confirm that the fragments generated were of the correct sequence. The oligonucleotide EDA001MO was covalently attached to a solid phase and used to capture the amplified product. Once captured the identity of the sequence was further confirmed by detection with the labelled probe EDA001B which was complementary to a sequence within the 129 bp fragment (Figure 6b).

## Claims

1. A method of amplifying complementary first and second nucleic acid sequences each of which has a binding region at its 3' end, the method comprising treating the separated single stranded sequences with
(a) first and second primers each capable of hybridising to the 3'-binding regions of the first and second strands respectively and each including remote from its 5'-end a digestion resistant region which, with the primer hybridized to the complementary 3-binding region, allows only partial digestion of the primer by the enzyme (d) having 5'-double strand specific exonuclease activity,
(b) third and fourth primers each having a degree of sequence homology with the partially digestible regions of the first and second primers respectively whereby the third and fourth primers are capable of hybridising to the 3'-binding regions of the first and second strands respectively,
(c) an enzyme having strand displacing polymerase activity,
(d) an enzyme having 5' double stranded specific exonuclease activity, said enzyme (d) possibly being provided by enzyme (c) in the case where the latter also has the required exonuclease activity, and
(e) nucleoside triphosphates,
under conditions permitting hybridisation, exonuclease digestion and strand displacement polymerisation thereby producing an amplified amount of the first and second strands.

2. A method as claimed in claim 1 wherein the digestion resistant region is provided by modified nucleotides or ribonucleotides.

3. A method as claimed in claim 2 wherein the modified nucleotides provide phosphorothiate linkages which provide the resistance to digestion by the exonuclease.

4. A method as claimed in any one of claims 1 to 3 wherein the first and second primers each comprise 30 to 60 bases.

5. A method as claimed in claim 4 wherein the digestion resistant region is provided 15 to 25 bases from the 5' end of the first and second primers.

6. A method as claimed in any one of claims 1 to 5 wherein the third primer is of a sequence corresponding to the sequence in the first primer on the 5' side of the digestion resistant region of that primer.

7. A method as claimed in any one of claims 1 to 6 wherein the fourth primer is of a sequence corresponding to the sequence in the second primer on the 5' side of the digestion resistant region of that primer.

8. A method as claimed in any one of claims 1 to 7 wherein the third and fourth primers comprise 12 to 30 bases.

9. A method as claimed in any one of claims 1 to 8 wherein the 5 double strand specific exonuclease is T7 Gene 6 exonuclease.

10. A method as claimed in any one of claims 1 to 9 wherein the strand displacing DNA polymerase is at least one of, 9°N polymerase, Klenow (exo⁻) polymerase, *Bst* polymerase, Vent (exo⁻) polymerase, or Deep Vent (exo⁻) polymerase, *Pfu* (exo⁻) polymerase, *Tth* polymerase, *Tfl* polymerase, *Taq* polymerase or *Bca* (exo⁻) polymerase.

11. A method as claimed in any one of claims 1 to 10 wherein the steps of exonuclease digestion and strand displacing polymerisation are effectively separated by performing the two reactions separately by removal of enzyme between steps, or, under conditions which favour the action of one or other enzyme.

12. A method as claimed in any one of claim 1 to 11 effected isothermally.

13. A method as claimed in anyone of claims 1 to 12 wherein the digestible regions of the first and second primers are of identical sequence and the third and fourth primers are identical to these sequences.

14. A method as claimed in anyone of claims 1 to 13 wherein the 5 -ends of the first, second, third and fourth primers have a degree of resistance to digestion whilst still leaving a digestible region of the primer.

15. A method as claimed in any one of claims 1 to 14 wherein the amplification occurs in the presence of further primers specific to other target sequences so as to provide multiplex amplification or to all or some of the same target sequence so as to provide nested amplification.

16. A method as claimed in any one of claims 1 to 15 wherein at least a portion of at least one of the nucleoside triphosphates provided as (e) of claim 1 is/are a modified such that when it is incorporated in a growing nucleic acid chain it is resistant to digestion by the exonuclease.

17. A method as claimed in any one of claims 1 to 16 wherein the nucleic acid is DNA.

## Patentansprüche

1. Verfahren zum Amplifizieren komplementärer erster und zweiter Nukleinsäuresequenzen, von denen jede eine Bindungsregion an ihrem 3'-Ende aufweist, wobei das Verfahren die Behandlung der getrennten einsträngigen Sequenzen mit
(a) ersten und zweiten Primern umfasst, wobei jeder zum Hybridisieren an den 3'-bindenden Regionen der ersten bzw. zweiten Stränge fähig ist und jeder entfernt von seinem 5'-Ende eine verdauungsresistente Region einschließt, die, mit dem Primer an die komplementäre 3'-bindende Region hybridisiert, nur einen teilweisen Verdau des Primers durch das Enzym (d) mit 5'-Doppelstrang-spezifischer Exonukleaseaktivität erlaubt,
(b) dritten und vierten Primern, wobei jeder einen Grad der Sequenzhomologie mit den teilweise verdaubaren Regionen der ersten bzw. zweiten Primer aufweist, wobei die dritten und vierten Primer zum Hybridisieren an den 3'-Bindungsregionen der ersten bzw. zweiten Stränge fähig sind,
(c) einem Enzym, das strangverdrängende Polymeraseaktivität aufweist,
(d) einem Enzym, das 5'-Doppelstrang-spezifische Exonukleaseaktivität aufweist, wobei das Enzym (d) möglicherweise durch Enzym (c) in dem Fall bereitgestellt wird, wenn das letztere auch die erforderliche Exonukleaseaktivität aufweist und
(e) Nukleosidtriphosphaten,
unter Bedingungen, die Hybridisierung, Exonuklease-Verdau und Strangverdrängungs-Polymerisierung erlauben, wodurch eine amplifizierte Menge der ersten und zweiten Stränge hergestellt wird.

2. Verfahren nach Anspruch 1, worin die verdauungsresistente Region durch modifizierte Nukleotide oder Ribonukleotide bereitgestellt wird.

3. Verfahren nach Anspruch 2, worin die modifizierten Nukleotide Phosphorothioat-Verknüpfungen bereitstellen, welche die Resistenz gegen Verdau durch die Exonuklease bereitstellen.

4. Verfahren nach einem der Ansprüche 1 bis 3, worin die ersten und zweiten Primer jeweils 30 bis 60 Basen umfassen.

5. Verfahren nach Anspruch 4, worin der verdauungsresistenten Region 15 bis 25 Basen vom 5'-Ende der ersten und zweiten Primer bereitgestellt werden.

6. Verfahren nach einem der Ansprüche 1 bis 5, worin der dritte Primer von einer Sequenz stammt, die der Sequenz im ersten Primer auf der 5'-Seite der verdauungsresistenten Region dieses Primers entspricht.

7. Verfahren nach einem der Ansprüche 1 bis 6, worin der vierte Primer von einer Sequenz ist, die der Sequenz im zweiten Primer auf der 5'-Seite der verdauungsresistenten Region dieses Primers entspricht.

8. Verfahren nach einem der Ansprüche 1 bis 7, worin die dritten und vierten Primer 12 bis 30 Basen umfassen.

9. Verfahren nach einem der Ansprüche 1 bis 8, worin die 5'-Doppelstrang-spezifische Exonuklease T7 Gen 6-Exonuklease darstellt.

10. Verfahren nach einem der Ansprüche 1 bis 9, worin die strangverdrängende DNS-Polymerase mindestens eine der folgenden darstellt: 9°N-Polymerase, Klenow-(exo⁻)-Polymerase, *Bst*-Polymerase, Vent-(exo⁻)-Polymerase oder Deep Vent-(exo⁻)-Polymerase, *Pfu*(exo^{*-*})-Polymerase, *Tth*-Polymerase, *Tfl*-Polymerase, *Taq*-Polymerase oder *Bca*-(exo⁻)-Polymerase.

11. Verfahren nach einem der Ansprüche 1 bis 10, worin die Schritte des Exonuklease-Verdaus und der Strangverdrängungs-Polymerisierung mittels Durchführen der beiden Reaktionen getrennt durch Entfernung des Enzyms zwischen den Schritten oder unter Bedingungen, welche die Wirkung von einem oder dem anderen Enzym begünstigen, wirksam getrennt werden.

12. Verfahren nach einem der Ansprüche 1 bis 11, das isothermisch bewirkt wird.

13. Verfahren nach einem der Ansprüche 1 bis 12, worin die verdaubaren Regionen der ersten und zweiten Primer eine identische Sequenz aufweisen und die dritten und vierten Primer mit diesen Sequenzen identisch sind.

14. Verfahren nach einem der Ansprüche 1 bis 13, worin die 5'-Enden der ersten, zweiten, dritten und vierten Primer einen Resistenzgrad gegen den Verdau aufweisen, während sie noch eine verdaubare Region des Primers zurücklassen.

15. Verfahren nach einem der Ansprüche 1 bis 14, worin die Amplifikation in Gegenwart weiterer Primer auftritt, die für andere Zielsequenzen spezifisch sind, um auf diese Weise eine Multiplex-Amplifikation bereitzustellen oder für alle oder einige der gleichen Zielsequenzen, um auf diese Weise eine verschachtelte (nested) Amplifikation bereitzustellen.

16. Verfahren nach einem der Ansprüche 1 bis 15, worin mindestens ein Teil von mindestens einem der Nukleosidtriphosphate wie unter (e) nach Anspruch 1 dergestalt modifiziert bereitgestellt ist/sind, dass es/sie gegen Verdau durch die Exonuklease resistent ist/sind, wenn es/sie in eine wachsende Nukleinsäurekette inkorporiert wird/werden.

17. Verfahren nach einem der Ansprüche 1 bis 16, worin die Nukleinsäure DNS darstellt.

## Revendications

1. Méthode d'amplification de première et deuxième séquences complémentaires d'acides nucléiques dont chacune a une région de liaison à son extrémité 3', la méthode comprenant traiter les séquences simple brin séparées avec
(a) des première et deuxième amorces capables chacune de s'hybrider aux régions de liaison 3' des premier et deuxième brins respectivement, et comprenant chacune une région éloignée de son extrémité 5', résistante à la digestion laquelle, avec l'amorce hybridée à la région de liaison 3' complémentaire, permet une digestion seulement partielle de l'amorce par l'enzyme (d) ayant une activité exonucléase 5' spécifique double brin,
(b) des troisième et quatrième amorces ayant un degré d'homologie de séquence avec les régions partiellement digestibles des première et deuxième amorces respectivement, en vertu de quoi les troisième et quatrième amorces sont capables de s'hybrider aux régions de liaison 3' des premier et deuxième brins respectivement,
(c) une enzyme ayant une activité polymérase de déplacement de brin,
(d) une enzyme ayant une activité exonucléase 5' spécifique double brin, ladite enzyme (d) étant peut-être fournie par l'enzyme (c) dans le cas où cette dernière possède aussi l'activité exonucléase requise, et
(e) des nucléoside-triphosphates,
dans des conditions permettant l'hybridation, la digestion par exonucléase et la polymérisation par déplacement de brin, produisant ainsi une quantité amplifiée des premier et deuxième brins.

2. Méthode telle que revendiquée dans la revendication 1, dans laquelle la région résistante à la digestion est fournie par des nucléotides ou des ribonucléotides modifiés.

3. Méthode telle que revendiquée dans la revendication 2, dans laquelle les nucléotides modifiés fournissent des liaisons phosphorothioate qui fournissent la résistance à la digestion par l'exonucléase.

4. Méthode telle que revendiquée dans l'une quelconque des revendications 1 à 3, dans laquelle les première et deuxième amorces comprennent chacune 30 à 60 bases.

5. Méthode telle que revendiquée dans la revendication 4, dans laquelle la région résistante à la digestion est fournie à 15-25 bases de l'extrémité 5' des première et deuxième amorces.

6. Méthode telle que revendiquée dans l'une quelconque des revendications 1 à 5, dans laquelle la troisième amorce est d'une séquence correspondant à la séquence dans la première amorce du côté 5' de la région résistante à la digestion de cette amorce.

7. Méthode telle que revendiquée dans l'une quelconque des revendications 1 à 6, dans laquelle la quatrième amorce est d'une séquence correspondant à la séquence dans la deuxième amorce du côté 5' de la région résistante à la digestion de cette amorce.

8. Méthode telle que revendiquée dans l'une quelconque des revendications 1 à 7, dans laquelle les troisième et quatrième amorces comprennent 12 à 30 bases.

9. Méthode telle que revendiquée dans l'une quelconque des revendications 1 à 8, dans laquelle l'exonucléase 5' spécifique double brin est l'exonucléase gène 6 de T7.

10. Méthode telle que revendiquée dans l'une quelconque des revendications 1 à 9, dans laquelle l'ADN polymérase de déplacement de brin est au moins l'une d'entre la 9°N polymérase, la Klenow (exo) polymérase, la *Bst* polymérase, la Vent (exo) polymérase ou la Deep Vent (exo) polymérase, la *Pfu* (exo) polymérase, la *Tht* polymérase, la *Tfl* polymérase, la *Taq* polymérase ou la *Bca* (exo) polymérase.

11. Méthode telle que revendiquée dans l'une quelconque des revendications 1 à 10, dans laquelle les étapes de digestion par exonucléase et de polymérisation par déplacement de brin, sont effectivement séparées en exécutant les deux réactions séparément en enlevant l'enzyme entre les étapes ou dans des conditions qui favorisent l'action d'une enzyme ou d'une autre.

12. Méthode telle que revendiquée dans l'une quelconque des revendications 1 à 11, effectuée de manière isothermique.

13. Méthode telle que revendiquée dans l'une quelconque des revendications 1 à 12, dans laquelle les régions digestibles des première et deuxième amorces sont de séquence identique et les troisième et quatrième amorces sont identiques à ces séquences.

14. Méthode telle que revendiquée dans l'une quelconque des revendications 1 à 13, dans laquelle les extrémités 5' des première, deuxième, troisième et quatrième amorces ont un degré de résistance à la digestion tout en laissant toujours une région digestible de l'amorce.

15. Méthode telle que revendiquée dans l'une quelconque des revendications 1 à 14, dans laquelle l'amplification a lieu en présence d'autres amorces spécifiques à d'autres séquences cibles de manière à donner une amplification multiplexe, ou à toute ou à une certaine partie de la même séquence cible de manière à donner une amplification nichée.

16. Méthode telle que revendiquée dans l'une quelconque des revendications 1 à 15, dans laquelle une partie au moins d'au moins l'un des nucléoside-triphosphates fournis comme dans (e) de la revendication 1, est/sont modifié/s de telle sorte que lorsqu'il/s est/sont incorporé/s dans une chaîne d'acide nucléique en croissance, il/s est/sont résistant/s à la digestion par l'exonucléase.

17. Méthode telle que revendiquée dans l'une quelconque des revendications 1 à 16, dans laquelle l'acide nucléique est de l'ADN.
